# EUROPÄISCHE PATENTANMELDUNG

(11) **EP 3 078 969 A2**
(43) Veröffentlichungstag der Anmeldung: **12.10.2016**
(21) Anmeldenummer: 16160833.6
(22) Anmeldetag: 17.03.2016
(51) Int. Cl.: G01N 33/543, G01N 33/487

(54) **DETEKTIONSVORRICHTUNG UND VERFAHREN ZUM DETEKTIEREN ZUMINDEST EINES ANALYTEN**

(30) Priorität: 30.03.2015 DE 102015205701
(71) Anmelder: ROBERT BOSCH GMBH, 70442 Stuttgart (DE)
(72) Erfinder: Hoffmann, Jochen, 71229 Leonberg (DE)

(57) **Zusammenfassung**

Die Erfindung betrifft eine Detektionsvorrichtung (100) zum Detektieren zumindest eines Analyten (135), wobei die Detektionsvorrichtung (100) eine Sensorkammer (105) mit einem darin angeordneten Sensor (120) zum Detektieren des Analyten (135) und zumindest eine fluidisch mit der Sensorkammer (105) gekoppelte oder koppelbare Trocknungsmittelkammer (110, 115) aufweist, wobei die Trocknungsmittelkammer (110, 115) ein Trocknungsmittel (170) aufweist, das ausgebildet ist, um einem das Trocknungsmittel (170) umgebenden Medium Feuchtigkeit zu entziehen.

## Beschreibung

### Stand der Technik

Die Erfindung betrifft eine Detektionsvorrichtung oder ein Verfahren zum Detektieren zumindest eines Analyten nach Gattung der unabhängigen Ansprüche. Gegenstand der vorliegenden Erfindung ist auch ein Computerprogramm.

Mikrofluidische Diagnosesysteme wie Lab-on-a-Chips (LoCs) erlauben die miniaturisierte und integrierte Durchführung komplexer fluidischer Arbeitsabläufe für den spezifischen Nachweis verschiedenster Moleküle. In publizierten LoC-Systemen für die DNA-Analytik wird das zu untersuchende Probenmaterial häufig mittels Polymerase-Kettenreaktion (PCR) amplifiziert und anschließend auf einem Mikroarray analysiert. Dabei werden die zu detektierenden Moleküle während der Amplifikation mit Fluoreszenzmolekülen markiert. Diese Moleküle binden dann an spezifische Bindungsstellen (Spots) auf dem Mikroarray und können dort beispielsweise fluorometrisch nachgewiesen werden. Die Fluoreszenzsignale eines Mikroarrays können mit Durch- oder Auflichtverfahren gemessen werden. Dabei hängt die Höhe der Signale maßgeblich von der Trockenheit der Fluorophor-beladenen Spots ab. Je feuchter die Fluoreszenzmoleküle sind, desto stärker werden die emittierten Signale reduziert. Werden Fluoreszenzmoleküle von einer Hydrathülle umgeben, gehen emittierte Fluoreszenzsignale über Schwingungsrelaxation an die Umgebung verloren. Im Stand der Technik wird on-Chip getrocknet in dem entweder Luft über das Mikroarray gepumpt wird oder Druckluft von extern in das LoC zum Trocknen eingeleitet wird. Dies ist mit den folgenden Nachteilen behaftet:
- Umpumpen von Luft on-Chip: Um Flüssigkeitströpfchen im Bereich des Arrays möglichst effizient mitzureißen bzw. Flüssigkeit in die Dampfphase zu überführen, wird ein starker Luftstrom benötigt. Aufgrund der in ihrem Fördervolumen begrenzten On-Chip-Pumplösungen sind solche Luftströme kaum zu erzeugen. Um eine effiziente Trocknung zu realisieren ist daher oft sehr lange zu trocknen (also zu pumpen). Ein weiteres Problem ist die Abfuhr der feuchten Luft. Wird lediglich On-Chip umgepumpt und geht dabei Feuchtigkeit in die Dampfhase über, wird die Luft in den Kanälen gesättigt und es kann keine weitere Feuchtigkeit mehr aufgenommen werden. Die Folge ist eine unzureichende Trockenwirkung.
- Einleiten externer Druckluft: Wird ein starker Luftstrom von extern zum Trocknen eines Mikroarrays in einem LoC verwendet, sollte der Luftstrom aus dem LoC herausgeführt und der Umgebungsluft zugeführt werden. Dies ist problematisch im Hinblick auf DNA- oder bakteriellen Kontaminationen entweder des Gerätes und/oder der Umgebungsluft.

### Offenbarung der Erfindung

Vor diesem Hintergrund werden mit dem hier vorgestellten Ansatz eine Detektionsvorrichtung, weiterhin ein entsprechendes Verfahren zum Detektieren sowie schließlich ein entsprechendes Computerprogramm gemäß den Hauptansprüchen vorgestellt. Durch die in den abhängigen Ansprüchen aufgeführten Maßnahmen sind vorteilhafte Weiterbildungen und Verbesserungen der im unabhängigen Anspruch angegebenen Vorrichtung möglich.

Der hier vorgestellte Ansatz schafft eine Detektionsvorrichtung zum Detektieren zumindest eines Analyten, wobei die Detektionsvorrichtung die folgenden Merkmale aufweist:
- eine Sensorkammer mit einem darin angeordneten Sensor zum Detektieren des Analyten; und
- zumindest eine fluidisch mit der Sensorkammer gekoppelte oder koppelbare Trocknungsmittelkammer, wobei die Trocknungsmittelkammer ein Trocknungsmittel aufweist, das ausgebildet ist, um einem das Trocknungsmittel umgebenden Medium Feuchtigkeit zu entziehen.

Unter einem (beispielsweise biochemischen) Analyten kann beispielsweise ein biologisches oder chemisches Material verstanden werden, dessen Auftreten mittels des Sensors erfasst werden soll. Beispielsweise kann ein solcher (biochemischer) Analyt ein DNA-Segment, ein Antigen, eine Umweltgefährdende Substanz oder dergleichen sein. Der Sensor kann beispielsweise ein Sensorträger, beispielsweise eine DNA-Mikroarray sein, auf dem ein oder mehrere Sensorelemente in der Form vom Spots aufgebracht oder befestigt sind, bei dem/denen sich beispielsweise ein Parameter durch eine Reaktion oder eine physikalische Wechselwirkung mit dem (biochemischen) Analyten verändert. Ein solcher Parameter kann beispielsweise eine optische Durchlässigkeit, Reflexionsfähigkeit oder eine Änderung der Farbe des Sensorelements bzw. Sensors sein, insbesondere wenn das Detektieren des (biochemischen) Analyten auf optische Weise erfolgen soll. Denkbar ist jedoch auch ein Wechsel des Parameters durch beispielsweise eine Änderung der elektrischen Leitfähigkeit oder der Kapazität dieses Sensors, insbesondere wenn das Detektieren des (biochemischen) Analyten auf elektrischem Wege erfolgen soll. Die Sensorkammer kann beispielsweise aus einem Kunststoffmaterial hergestellt sein. Die Trocknungsmittelkammer kann ebenfalls aus einem Kunststoffmaterial hergestellt sein, günstigerweise aus dem gleichen Kunststoffmaterial wie die Sensorkammer. Dabei können die Sensorkammer und die Trocknungsmittelkammer auch einstückig, insbesondere als integriertes Bauteil ausgestaltet sein. Die Sensorkammer und die Trocknungsmittelkammer sind dabei fluidisch gekoppelt oder koppelbar. Hierbei kann beispielsweise ein Ventil vorgesehen sein, um den Durchgang von einem Fluid von der Sensorkammer in die Trocknungsmittelkammer oder umgekehrt freizugeben oder zu unterbinden. Unter einem Trocknungsmittel kann ein Stoff verstanden werden, der durch die chemische Reaktion oder physikalische Bindung Feuchtigkeit, insbesondere Wasser oder Wasserdampf, aus einem Medium wie einem Fluid, insbesondere einem Gas, ein Gasgemisch oder ein Aerosol, bindet. Dieses Medium ist vorzugsweise in unmittelbarem Kontakt mit dem Trocknungsmittel, wenn das Trocknungsmittel dem Medium die Feuchtigkeit entzieht. Dieses Medium kann beispielsweise ein Fluid, speziell ein Gas, ein Gasgemisch oder ein Aerosol sein, welches von der Sensorkammer in die Trocknungsmittelkammer oder umgekehrt verlagert werden kann.

Der hier vorgestellte Ansatz basiert auf der Erkenntnis, dass durch die Verwendung eines speziellen Trocknungsmittels in einer separaten Trocknungsmittelkammer sehr effizient der (biochemische) Analyt auf dem Sensor detektiert werden kann. Durch den Entzug der Feuchtigkeit mittels der Trocknung mittels des Trocknungsmittels kann somit eine Anlagerung von Wasser- oder Wasserdampfmolekülen an Sensorelementen mit gebundenen oder angelagerten Partikeln des (biochemischen) Analyten verhindert werden, die eine Detektion dieser Partikel des (biochemischen) Analyten verhindert oder erschwert. Zugleich ist die technische Umsetzung des hier vorgestellten Ansatzes sehr einfach und mit geringen Kosten verbunden, sodass sich der hier vorgestellte Ansatz für eine Detektionsvorrichtung oder ein Verfahren zum Detektieren für einen Masseneinsatz gut geeignet.

Vorteilhaft ist eine Ausführungsform des hier vorgestellten Ansatzes, bei dem die Sensorkammer mittels zumindest eines schaltbaren Ventils fluidisch mit der Trocknungsmittelkammer gekoppelt oder koppelbar ist. Eine solche Ausführungsform des hier vorgestellten Ansatzes bietet den Vorteil, dass das Vorsehen eines solchen Ventils einerseits technisch sehr einfach zu realisieren ist und andererseits (insbesondere bei geschlossenem Zustand des Ventils) vorteilhaft die Aufrechterhaltung der Funktionsfähigkeit des Trocknungsmittels über einen langen Zeitraum ermöglicht.

Gemäß einer weiteren Ausführungsform des hier vorgestellten Ansatzes ist eine flexibel verformbare Abdeckmembran vorgesehen, die die zumindest eine Trocknungsmittelkammer und/oder die Sensorkammer fluidisch gegenüber einer Außenumgebung der Detektionsvorrichtung verschließt. Eine solche Ausführungsform des hier vorgestellten Ansatzes bietet den Vorteil, durch Niederdrücken dieser Abdeckmembran technisch sehr einfach das Medium aus oder in die Trocknungsmittelkammer zu fördern. Dies kann beispielsweise durch einen Aktor erfolgen, der von externen auf diese Abdeckmembran drückt und hierbei das Volumen in der Sensorkammer und/oder der Trocknungsmittelkammer verringert und hierdurch das Medium in der entsprechenden Kammer ausfördert.

Einer weiteren Ausführungsform des hier vorgestellten Ansatzes kann die Sensorkammer zumindest einen mittels eines Einlassventils verschließbaren Einlassanschluss und/oder einen mittels eines Auslassventils verschließbaren Auslassanschluss aufweisen, wobei der (biochemische) Analyt zumindest durch den Einlassanschluss in die Sensorkammer zuführbar und/oder durch den Auslassanschluss abführbar ist. Eine solche Ausführungsform des hier vorgestellten Ansatzes bietet den Vorteil, den Sensor erst unmittelbar vor der Benutzung gegenüber einer Umwelt freizugeben und hiermit die Aufrechterhaltung der Funktionsfähigkeit des Sensors bzw. des Materials des Sensors über einen langen Zeitraum zu ermöglichen.

Um eine möglichst effiziente Trocknung eines Mediums in der Sensorkammer zu erreichen, kann gemäß einer günstigen Ausführungsform des hier vorgestellten Ansatzes die Trocknungsmittelkammer fluidisch zwischen das Einlassventil und die Sensorkammer und/oder zwischen das Auslassventil und die Sensorkammer geschaltet sein. Auf diese Weise ist es möglich, ein Medium nach Einlass in die Sensorkammer und einem Verschließen beispielsweise des Einlass- und/oder Auslassventils dennoch mit dem Trocknungsmittel in Kontakt bringen zu können, um diesem Medium Feuchtigkeit zu entziehen und hierdurch eine Detektion des (biochemischen) Analyten zu ermöglichen oder zu verbessern.

Besonders vorteilhaft ist ferner eine Ausführungsform des hier vorgestellten Ansatzes, bei dem zumindest eine fluidisch mit der Sensorkammer gekoppelte oder koppelbare zweite Trocknungsmittelkammer vorgesehen ist, wobei die zweite Trocknungsmittelkammer ein zweites Trocknungsmittel aufweist, das ausgebildet ist, um einem das zweite Trocknungsmittel umgebenden Medium Feuchtigkeit zu entziehen. Eine solche Ausführungsform des hier vorgestellten Ansatzes bietet den Vorteil einer besonders effizienten Trocknung des Mediums, welches in die Sensorkammer verlagert werden soll, da für eine solche Trocknung die Trocknungsmittel aus zwei Trocknungsmittelkammern zur Verfügung stehen. Diese Trocknungsmittelkammern lassen sich technisch sehr einfach in einer entsprechenden Detektionsvorrichtung bereitstellen.

Besonders einfach lässt sich das Medium von der Sensorkammer in die Trocknungsmittelkammer oder die zweite Trocknungsmittelkammer oder aber von der Trocknungsmittelkammer in die zweite Trocknungsmittelkammer verlagern, wenn die zweite Trocknungsmittelkammer auf einer der Trocknungsmittelkammer gegenüberliegenden Seite der Sensorkammer angeordnet ist. In diesem Fall kann ein Aktor sehr einfach ausgestaltet werden, indem lediglich das Medium in eine Richtung gedrückt oder verschoben werden soll.

Denkbar ist ferner auch eine Ausführungsform des hier vorgestellten Ansatzes, bei dem die Sensorkammer fluidisch zwischen der Trocknungsmittelkammer und der zweiten Trocknungsmittelkammer angeordnet ist. Auch hier lässt sich technisch sehr einfach das Medium oder Fluid von der Sensorkammer in die Trocknungsmittelkammer oder die zweite Trocknungsmittelkammer oder umgekehrt verlagern, sodass eine effiziente Trocknung dieses Mediums möglich wird.

Gemäß einer weiteren Ausführungsform des hier vorgestellten Ansatzes kann die Trocknungsmittelkammer fluidisch zur Sensorkammer parallel geschaltet oder parallel schaltbar sein. Eine solche Ausführungsform des hier vorgestellten Ansatzes bietet den Vorteil, das Medium sehr effizient durch die Trocknungsmittelkammer und die Sensorkammer führen zu können, indem beispielsweise eine entsprechende Pumpeinrichtung lediglich das Medium in eine Richtung pumpen soll und somit das Medium in einem Kreislauf zwischen der Trocknungsmittelkammer und der Sensorkammer bewegt werden kann.

Technisch besonders einfach herstellbar ist eine Detektionsvorrichtung gemäß einer Ausführungsform der vorliegenden Erfindung, bei der ein Gehäuse vorgesehen ist, in dem die zumindest eine Trocknungsmittelkammer und die Sensorkammer ausgebildet sind. Eine solche Ausführungsform des hier vorgestellten Ansatzes bietet den Vorteil einer technisch einfachen und kostengünstigen Herstellung eines (beispielsweise Kunststoff-) Blisters, in dem die Detektionsvorrichtung ausgebildet werden kann.

Gemäß einer weiteren Ausführungsform des hier vorgestellten Ansatzes kann die Trocknungsmittelkammer das Trocknungsmittel in pulver- oder kugelförmiger Form aufweisen. Eine solche Ausführungsform des hier vorgestellten Ansatzes bietet den Vorteil, dass ein solches Trocknungsmittel sehr stabil in einer bestimmten Position der Trocknungsmittelkammer fixiert werden kann, sodass eine möglichst gute Funktion dieses Trocknungsmittels sichergestellt werden kann.

Günstig ist ferner eine Ausführungsform des hier vorgestellten Ansatzes als Verfahren zum Detektieren zumindest eines Analyten unter Verwendung einer Detektionsvorrichtung gemäß einem hier vorgestellten Ansatz, wobei das Verfahren die folgenden Schritte aufweist:
- Einleiten oder Aufnehmen des Analyten in die Sensorkammer; und
- Pumpen eines Fluids von der Sensorkammer in die Trocknungsmittelkammer und/oder eines Fluids von der Trocknungsmittelkammer in die Sensorkammer.

Dieses Verfahren kann beispielsweise in Software oder Hardware oder in einer Mischform aus Software und Hardware beispielsweise in einem Steuergerät implementiert sein.

Der hier vorgestellte Ansatz schafft ferner ein Steuergerät, das ausgebildet ist, um die Schritte einer Variante eines hier vorgestellten Verfahrens in entsprechenden Einrichtungen durchzuführen, anzusteuern bzw. umzusetzen. Auch durch diese Ausführungsvariante der Erfindung in Form eines Steuergeräts kann die der Erfindung zugrunde liegende Aufgabe schnell und effizient gelöst werden.

Unter einem Steuergerät kann vorliegend ein elektrisches Gerät verstanden werden, das Sensorsignale verarbeitet und in Abhängigkeit davon Steuer- und/oder Datensignale ausgibt. Das Steuergerät kann eine Schnittstelle aufweisen, die hard- und/oder softwaremäßig ausgebildet sein kann. Bei einer hardwaremäßigen Ausbildung können die Schnittstellen beispielsweise Teil eines sogenannten System-ASICs sein, der verschiedenste Funktionen des Steuergeräts beinhaltet. Es ist jedoch auch möglich, dass die Schnittstellen eigene, integrierte Schaltkreise sind oder zumindest teilweise aus diskreten Bauelementen bestehen. Bei einer softwaremäßigen Ausbildung können die Schnittstellen Softwaremodule sein, die beispielsweise auf einem Mikrocontroller neben anderen Softwaremodulen vorhanden sind.

Von Vorteil ist auch ein Computerprogrammprodukt oder Computerprogramm mit Programmcode, der auf einem maschinenlesbaren Träger oder Speichermedium wie einem Halbleiterspeicher, einem Festplattenspeicher oder einem optischen Speicher gespeichert sein kann und zur Durchführung, Umsetzung und/oder Ansteuerung der Schritte des Verfahrens nach einer der vorstehend beschriebenen Ausführungsformen verwendet wird, insbesondere wenn das Programmprodukt oder Programm auf einem Computer oder einer Vorrichtung ausgeführt wird.

Ausführungsbeispiele der Erfindung sind in den Figuren dargestellt und in der nachfolgenden Beschreibung näher erläutert. Es zeigt:
Fig. 1A ein Blockschaltbild einer Detektionsvorrichtung gemäß einem ersten Ausführungsbeispiel;
Fig. 1B ein Blockschaltbild einer Detektionsvorrichtung gemäß einem weiteren Ausführungsbeispiel;
Fig. 2 ein Blockschaltbild einer Detektionsvorrichtung gemäß einem weiteren Ausführungsbeispiel;
Fig. 3 ein Blockschaltbild einer Detektionsvorrichtung gemäß einem weiteren Ausführungsbeispiel; und
Fig. 4 ein Ablaufdiagramm eines Verfahrens gemäß einem Ausführungsbeispiel.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

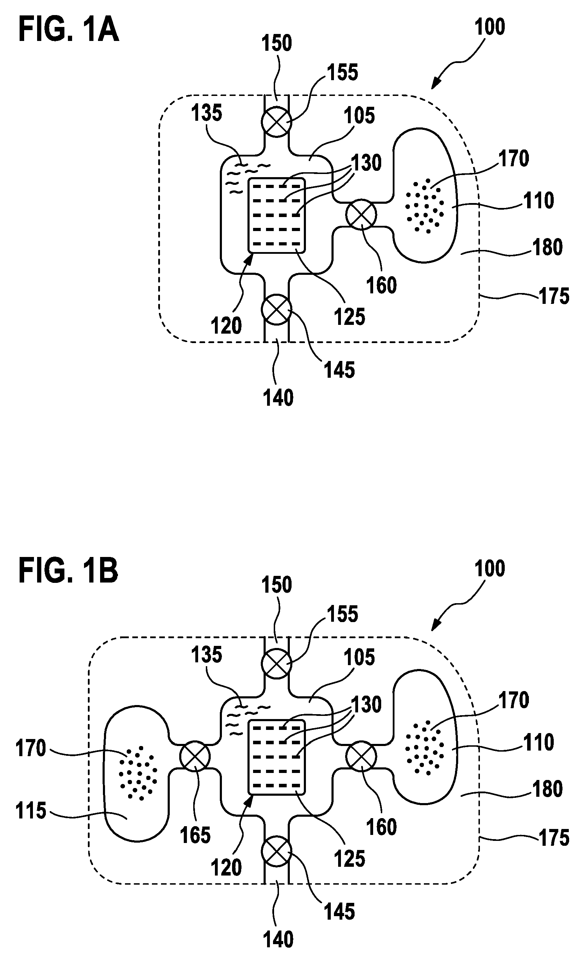

Fig. 1A zeigt ein Blockschaltbild einer Detektionsvorrichtung 100 gemäß einem ersten Ausführungsbeispiel in Aufsichtdarstellung. Die Detektionsvorrichtung 100 weist eine Sensorkammer 105 und eine erste Trocknungsmittelkammer 110 auf. In der Sensorkammer 105 (die beispielsweise ein Volumen von 5 bis 500 µl aufweist) ist ein Sensor 120 angeordnet, der beispielsweise in der Form eines Sensorträgers 125 mit darauf fixierten Sensorelementen 130 vorliegt. Dieser Sensor 120 kann somit als Mikroarray verstanden werden, der in der Sensorkammer 105 als einer Mikroarray-Kammer fixiert ist. In dieser Sensorkammer 105 kann eine biochemische Reaktion zwischen einem Analyten 135 (der beispielsweise DNA oder Antigene enthält bzw. daraus besteht) mit Fängermolekülen (beispielsweise als DNA-Sonden oder Antikörper, die auf den Sensorelementen 130 fixiert sind oder die die Sensorelemente 130 bilden) stattfinden. Um den Analyten 135 mit dem Sensor 120 in Kontakt zu bringen, wird dieser Analyt durch den Einlassanschluss 140 nach Öffnung des Einlassventils 145 in die Sensorkammer 105 eingelassen. Um ein schnelles Füllen der Sensorkammer 105 zu ermöglichen, kann ferner auch ein Auslassanschluss 150 mit einem Auslassventil 155 vorgesehen sein, sodass der Analyt bei geöffnetem Einlassventil 145 und Auslassventil 155 über den Einlassanschluss 140 durch die Sensorkammer 105 und den Auslassanschluss 150 geführt werden kann. In diesem Fall wird der Analyt mit den Sensorelementen 130 in Kontakt gebracht, wobei bei dem tatsächlichen Vorliegen des Analyten 135 eine chemische Reaktion mit Sensorelementen 130 erfolgen kann, die zu einer Veränderung der optischen Eigenschaften des Sensorelementes 130 führt, sodass das Vorhandensein des Analyten 135 durch eine optische Auswertung des Sensorelementes bzw. des Sensors 120 erfolgen kann.

Die erste Trocknungsmittelkammer 110 (die beispielsweise ein Volumen von 10 µl bis 5 ml, bevorzugt ein Volumen von 25 µl bis 1 ml, aufweist) ist durch ein erstes Trocknungsmittelkammerventil 160 mit der Sensorkammer 105 fluidisch gekoppelt oder koppelbar. In der ersten Trocknungsmittelkammer 110 ist ein Trocknungsmittel 170. Als Trocknungsmittel 170 eignen sich prinzipiell alle Mittel, die zu einem Entzug von Feuchtigkeit aus einem Medium, insbesondere einem Gas geeignet sind. Beispielhaft für die Wahl eines Materials eines Trocknungsmittels 170 lassen sich die folgenden Materialien nennen:
Chemische Trocknungsmitte:
   Phosphorpentoxid, Aluminiumoxid, Calcium, Caldumhydrid, Calciumoxid, Calciumsulfat, Kaliumcarbonat, Kaliumhydroxid, Kupfersulfat, Lithiumaluminiumhydrid, Natriumhydroxid, Silicagel, Blaugel, Orangegel, Zeolithe, Natriumsulfat, Magnesiumsulfat;

Adsorptive Trocknungsmitte:
Molekularsieb, Bentonit;

Das Trocknungsmittel 170 kann als Pulver oder kugelförmiges Granulat vorliegen und an fest vordefinierten Positionen in den jeweiligen Trocknungsmittelkammern 110 fixiert sein. Auf diese Weise kann eine möglichst optimale Anordnung des Trocknungsmittels 170 in den Trocknungsmittelkammern 110 sichergestellt werden, damit das Trocknungsmittel möglichst dem Medium (hier beispielsweise Luft) viel Feuchtigkeit entziehen kann.

Fig. 1B zeigt ein Blockschaltbild einer Detektionsvorrichtung gemäß einem weiteren Ausführungsbeispiel der vorliegenden Erfindung. Im Unterschied zum Ausführungsbeispiel aus Fig. 1A ist nun auf einer der ersten Trocknungsmittelkammer 110 bezüglich der Sensorkammer 105 gegenüberliegende Seite eine zweite Trocknungsmittelkammer 115 vorgesehen. Die zweite Trocknungsmittelkammer 115 (die ebenfalls beispielsweise ein Volumen von 10 µl bis 5 ml, bevorzugt ein Volumen von 25 µl bis 1 ml, aufweist) ist durch ein Trocknungsmittelkammerventil 165 mit der Sensorkammer 105 fluidisch gekoppelt oder koppelbar. Als Trocknungsmittel 170 in der zweiten Trocknungsmittelkammer 115 kommen ebenfalls die mit Bezug zur ersten Trocknungsmittelkammer 110 genannten Materialen und deren Befestigung in der zweiten Trocknungsmittelkammer 115 in Betracht, insbesondere ist das Trocknungsmittel 170 in der zweiten Trocknungsmittelkammer 115 im vorliegenden Ausführungsbeispiel identisch zu dem Trocknungsmittel 170 in der ersten Trocknungsmittelkammer 110. Denkbar ist jedoch auch ein Ausführungsbeispiel, bei dem das Trocknungsmittel in der der ersten

Trocknungsmittelkammer 110 unterschiedlich von dem Trocknungsmittel in der zweiten Trocknungsmittelkammer ist (beispielsweise in der ersten Trocknungsmittelkammer 110 ein chemisches Trocknungsmittel, während in der zweiten Trocknungsmittelkammer 115 ein adsorptives Trocknungsmittel enthalten ist.

Um nun nach dem Einleiten des (biochemischen) Analyten den Sensor auslesen zu können, sollten vor dem Auslesen des Mikroarrays bzw. Sensors 120 ungebundene Analyten weggewaschen werden. Hierbei sind je das Einlassventil 145 und Auslassventil 155 geöffnet, während die Ventile 160 und 165 zu der ersten und zweiten Trocknungsmittelkammer 110 bzw. 115 geschlossen sind.

Um das Mikroarray bzw. den Sensor 120 jetzt effizient auszulesen bzw. ein solches Auslesen vorzubereiten, werden das Einlassventil 145 und das Auslassventil 155 geschlossen um das zu trocknende Volumen auf die Mikroarray-Kammer bzw. Sensorkammer 105 zu beschränken. Zusätzlich werden die Ventile 160 und 165 zu der ersten und zweiten Trocknungsmittelkammer 110 bzw. 115 geöffnet, wodurch eine (fluidisch durchlässige) Verbindung zwischen den das Trocknungsmittel 170 enthaltende Trocknungsmittelkammern 110 und 115 und der Mikroarray-Kammer bzw. Sensorkammer 105 hergestellt wird. 1 - 95 Volumenprozent der Trocknungsmittelkammern sind mit einem Trockenmittel gefüllt, wie es beispielsweise vorstehend genannt wurde. Der Chip als Detektionsvorrichtung 100 ist aus einem polymeren Mehrschichtverbund als Gehäuse 175 aufgebaut, bei dem eine direkt an das fluidische System angrenzende Schicht aus einer auslenkbaren (Abdeck-) Membran 180 besteht. Diese Abdeckmembran 180 kann beispielsweise die als Ausnehmungen in dem Gehäuse 175 ausgebildete die Sensorkammer 105, erste und/oder die zweite Trocknungsmittelkammer 110 und 115 überdecken und hierdurch fluidisch von einer Außenumgebung der Detektionsvorrichtung 100 abkapseln. Lediglich durch den Einlassanschluss 140 und den Auslassanschuss 150 ist dann der Analyt mit dem (biochemischen) Analyten 135 in die Sensorkammer 105 zuführbar. Durch Auslenken dieser (Abdeck-) Membran 180 können Volumina in Kammern 105, 110 und 115 verdrängt werden. Ein in Fig. 1 von rechts nach links (oder umgekehrt) verlaufender Luftstrom (von Luft als Medium) kann durch die regelmäßige Auslenkung/Aktuierung der (Teil-) Membranen der Membran 175 über den Trocknungsmittelkammern 110 und 115 oder der Sensorkammer 105 erzeugt werden. Dieses Auslenken bzw. Aktuieren kann beispielsweise mit einer antizyklischen Aktuierung bzw. einem antizyklischen Niederdrücken der Membran 180 auf den Trockenmittelkammern 110 bzw. 115 oder die Sensorkammer 105 erfolgen, wie dies beispielsweise sehr einfach durch einen Aktor in der Form einer Aktuator-Wippe oder eines piezoelektrischen Aktors realisiert werden kann. Denkbar ist jedoch auch ein Ausführungsbeispiel der vorliegenden Erfindung, bei der auch lediglich eine oder zwei Kammern 105, 110 bzw. 115 von dieser Abdeckmembran 1680 bedeckt sind, um die Förderung von Fluid zwischen zwei von diesen Kammern 105, 110 bzw. 115 zu bewerkstelligen.

In Fig. 1 sind beide Trocknungsmittelkammern 110 und 115 mit Trocknungsmittel 170 befüllt. Es ist aber auch vorstellbar, dass nur eine der bei den Kammern mit Trocknungsmittel 170 befüllt ist. Denkbar ist auch ein Ausführungsbeispiel, bei dem nur eine Trocknungsmittelkammer vorhanden ist.

Ein wichtiger Aspekt des hier vorgestellten Ansatzes ist die Integration eines Trocknungsmittels 170 in einer speziellen Anordnung zu der den Sensor 120 bzw. das DNA-Mikroarray beinhaltenden (Sensor-) Kammer 105. Zum Trocknen eines DNA-Mikroarrays bzw. Sensors 120 nach der Hybridisierung wird ein Luftstrom in der Mikroarraykammer bzw. Sensorkammer 105 erzeugt. Dabei nimmt die strömende Luft Feuchtigkeit der Mikroarray-Kammer bzw. Sensorkammer 105 auf und gibt diese an ein in dem Strömungskreislauf vorhandenes Trocknungsmittel 170 wie das in den Trocknungsmittelkammern 110 bzw. 115 angeordnete Trocknungsmittel wieder ab.

Durch ein solches Vorgehen ergeben sich mehrere Vorteile für die Detektion des (biochemischen) Analyten:
- Es ist kein Trocknen mittels eines externen Luftstroms notwendig. Dadurch gelangen keine amplifizierten DNA-Moleküle oder Pathogene in das Gerät bzw. die Detektionsvorrichtung 100 bzw. die Laboraußenluft, wodurch die Gefahr von falsch-positiven Signalen bzw. Gefährdungen des Bedieners reduziert werden kann.
- Durch das Vorsehen von entsprechenden Ventilen in günstigen Ausführungsformen kann die Mikroarray-Kammer bzw. Sensorkammer 105 und die Trocknungsmittel-Kammer(n) 110 bzw. 115 von der restlichen Fluidik fluidisch entkoppelt werden, wodurch sich das zu entfeuchtende Volumen reduziert und das Mikroarray bzw. der Sensor 120 (insbesondere die darauf angeordneten Sensorelemente 130) schneller getrocknet und dadurch schneller ausgelesen werden kann.
- Das gleiche Trockenmittel 170 kann auch verwendet werden, um während einer Lagerperiode der Kartusche die Bioreagenzien bzw. Sensorelemente 130 vor Feuchtigkeit und damit Degradation zu schützen. Während der Prozessierung einer Kartusche als Detektionsvorrichtung 100 könnten beispielsweise die das Trockenmittel 170 enthaltenden Kammern 110 bzw. 115 mittels Ventilen 160 bzw. 165 vor Feuchtigkeit abschirmen und die Trockenmittelkammer 110 bzw. 115 erst zum Trocknen des Mikroarrays bzw. Sensors 120 mit dem entsprechenden Pfad fluidisch verschalten.
- Es kann eine Einbringung des Trocknungsmittels 170 in Form kleiner Kügelchen erfolgen. Die Form des Trocknungsmittels 170 bzw. der Einbringung ermöglicht ein präzises und einfaches Einbringen und Fixieren des Trocknungsmittels 170 in den jeweiligen Trocknungsmittelkammern 110 bzw. 115, wodurch sich die Entfeuchtung weiter verbessern lässt, insbesondere wenn das Trocknungsmittel 170 von möglichst vielen Seiten her zugänglich ist.

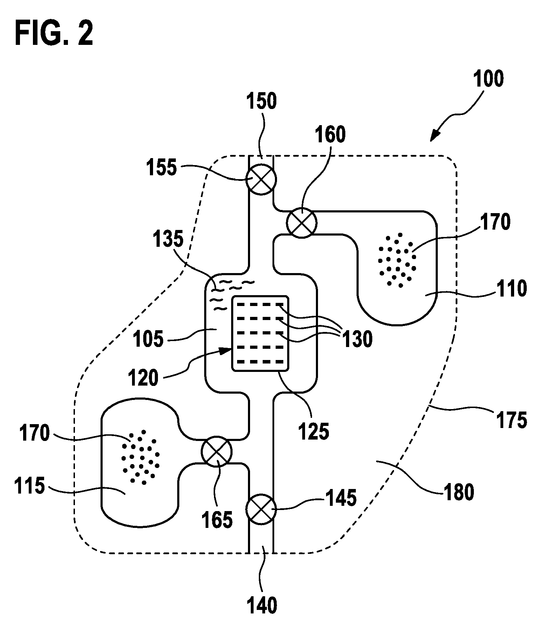

Fig. 2 zeigt Blockschaltbild einer Detektionsvorrichtung 100 gemäß einem zweiten Ausführungsbeispiel in einer Aufsichtdarstellung. Hierbei sind die Trocknungsmittelkammern 110 und 115 nicht direkt an die Mikroarray-Kammer bzw. Sensorkammer 105 angeschlossen, sondern mit den an die Mikroarray-Kammer bzw. Sensorkammer 105 angeschlossenen fluidischen Kanälen, d. h. den Einlassanschluss 140 und den Auslassanschluss 150 verbunden. Das Ausführungsbeispiel gemäß Fig. 2 hat gegenüber der dem Ausführungsbeispiel gemäß Fig. 1 den Vorteil, dass sich der in den an die Trocknungsmittelkammer 110 bzw. 115 angeschlossenen Kanälen induzierte Luftstrom beim Eintritt in die Mikroarray-Kammer bzw. Sensorkammer 105 nicht so starke aufweitet (wobei eine Reduktion der Strömungsgeschwindigkeit resultieren würde) und damit verbunden die Trockenwirkung reduziert wird. Es werden auch keine Turbulenzen induziert, da die Form der Sensorkammer 105 beispielsweise linsenförmig ausgestaltet werden kann und somit für eine Fluiddurchströmung optimal ausgelegt werden kann. In diesem Ausführungsbeispiel ist also ein höherer bzw. schnellerer Luftstrom und damit verbunden eine bessere Trockenwirkung realisierbar. Als weiterer Vorteil wird das Strömungsprofil / durch die Mikroarray-Kammer bzw. Sensorkammer 105 hindurch nicht wie dem Ausführungsbeispiel nach Fig. 1 durch seitlich einmündende Kanäle zu den Trocknungsmittelkammern 110 bzw. 115 beeinflusst.

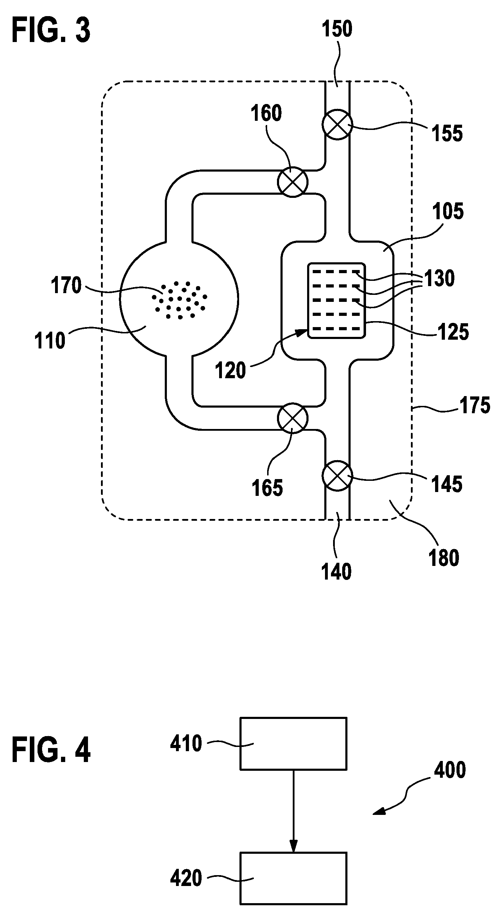

Fig. 3 zeigt das Blockschaltbild einer Detektionsvorrichtung 100 gemäß einem weiteren Ausführungsbeispiel in einer Aufsichtsdarstellung. Hierbei ist das Trocknungsmittel 170 in eine Trocknungsmittelkammer 110 eingebracht, die von den beiden Trocknungsmittelkammerventilen 160 bzw. 165 begrenzt ist. Die beiden Trocknungsmittelkammerventile 160 und 165 sind hier als Pumpventile ausgestaltet, wogegen die (nun einzige) Trocknungsmittelkammer 110 der Sensorkammer 105 parallel geschaltet ist und als Pumpkammer wirkt. Wie auch in den in den Figuren 1 und 2 dargestellten Ausführungsbeispielen werden die Ventile 145 und 155 geschlossen, um das zu trocknende Volumen auf die Mikroarray-Kammer bzw. Sensorkammer 105 zu beschränken. Anschließend werden alternierend die Trocknungsmittelkammerventile 160 und 165 und die als Pumpkammer wirkende Trocknungsmittelkammer 110 beispielsweise in einer dem Fachmann bekannten Art und Weise (beispielsweise als semistatische Pumpe oder unter Zuhilfenahme von Druckluft zum Niederdrücken der Membran 180 über der Trocknungsmittelkammer 110 oder der Sensorkammer 105 aktuiert, um einen Luftstrom durch die Mikroarray-Kammer bzw. Sensorkammer 105 zu leiten. Dies hat gegenüber den vorstehend beschriebenen Ausführungsbeispielen gemäß den Figuren 1 und 2 den Vorteil, dass nur eine Kammer als Pumpkammer benötigt wird und damit verbunden die Trocknungseinrichtung auf dem Chip bzw. der Detektionsvorrichtung 100 weniger Platz benötigt.

Fig. 4 zeigt ein Ablaufdiagramm eines Ausführungsbeispiels als Verfahren 400 zum Detektieren zumindest eines (biochemischen) Analyten. Das Verfahren 400 nutzt hierzu eine Detektionsvorrichtung 100 gemäß einem vorstehend näher beschrieben Ausführungsbeispiel. Das Verfahren 400 umfasst einen Schritt 410 des Einleitens oder Aufnehmens des (biochemischen) Analyten in die Sensorkammer und einen Schritt 420 des Pumpens eines Mediums von der Sensorkammer in die Trocknungsmittelkammer und/oder eines Mediums von der Trocknungsmittelkammer in die Sensorkammer, insbesondere um dem Medium Feuchtigkeit zu entziehen. Zusätzlich kann auch nach dem Schritt 410 des Einleitens oder Aufnehmens und vor dem Schritt 420 des Pumpens noch ein Spülen der Sensorkammer mit einem Waschpuffer und/oder oder ein Ausfördern von nicht an Sensorelementen in der Sensorkammer gebundenen Analyten aus der Sensorkammer erfolgen. Hierdurch wird eine Aufbereitung der Auslesung des Sensors optimiert.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Detektionsvorrichtung (100) zum Detektieren zumindest eines Analyten (135), wobei die Detektionsvorrichtung (100) die folgenden Merkmale aufweist:
- eine Sensorkammer (105) mit einem darin angeordneten Sensor (120) zum Detektieren des Analyten (135); und
- zumindest eine fluidisch mit der Sensorkammer (105) gekoppelte oder koppelbare Trocknungsmittelkammer (110, 115), wobei die Trocknungsmittelkammer (110, 115) ein Trocknungsmittel (170) aufweist, das ausgebildet ist, um einem das Trocknungsmittel (170) umgebenden Medium Feuchtigkeit zu entziehen.

2. Detektionsvorrichtung (100) gemäß Anspruch 1, **dadurch gekennzeichnet, dass** die Sensorkammer (105) mittels zumindest eines schaltbaren Ventils (160, 165) fluidisch mit der Trocknungsmittelkammer (110, 115) gekoppelt oder koppelbar ist.

3. Detektionsvorrichtung (100) gemäß einem der vorangegangenen Ansprüche, **gekennzeichnet, durch** eine flexibel verformbare Abdeckmembran (180), die die zumindest eine Trocknungsmittelkammer (110, 115) und/oder die Sensorkammer (105) fluidisch gegenüber einer Außenumgebung der Detektionsvorrichtung (100) verschließt.

4. Detektionsvorrichtung (100) gemäß einem der vorangegangenen Ansprüche, **dadurch gekennzeichnet, dass** die Sensorkammer (105) zumindest einen mittels eines Einlassventils (145) verschließbaren Einlassanschluss (140) und/oder einen mittels eines Auslassventils (155) verschließbaren Auslassanschluss (150) aufweist, wobei der Analyt (135) zumindest durch den Einlassanschluss (140) in die Sensorkammer (105) zuführbar und/oder durch den Auslassanschluss (150) abführbar ist.

5. Detektionsvorrichtung (100) gemäß Anspruch 4, **dadurch gekennzeichnet, dass** die Trocknungsmittelkammer (110, 115) fluidisch zwischen das Einlassventil (145) und die Sensorkammer (105) und/oder zwischen das Auslassventil (155) und die Sensorkammer (105) geschaltet ist.

6. Detektionsvorrichtung (100) gemäß einem der vorangegangenen Ansprüche, **gekennzeichnet, durch** zumindest eine fluidisch mit der Sensorkammer (105) gekoppelte oder koppelbare zweite Trocknungsmittelkammer (115), wobei die zweite Trocknungsmittelkammer (115) ein zweites Trocknungsmittel (170) aufweist, das ausgebildet ist, um einem das zweite Trocknungsmittel (170) umgebendes Medium Feuchtigkeit zu entziehen.

7. Detektionsvorrichtung (100) gemäß Anspruch 6, **dadurch gekennzeichnet, dass** die zweite Trocknungsmittelkammer (115) auf einer der Trocknungsmittelkammer (110) gegenüberliegenden Seite der Sensorkammer (105) angeordnet ist.

8. Detektionsvorrichtung (100) gemäß einem der Ansprüche 6 oder 7, **dadurch gekennzeichnet, dass** die Sensorkammer (105) fluidisch zwischen der Trocknungsmittelkammer (115) und der zweiten Trocknungsmittelkammer (110, 115) angeordnet ist.

9. Detektionsvorrichtung (100) gemäß einem der vorangegangenen Ansprüche, **dadurch gekennzeichnet, dass** die Trocknungsmittelkammer (110, 115) fluidisch zur Sensorkammer (105) parallel geschaltet oder parallel schaltbar ist.

10. Detektionsvorrichtung (100) gemäß einem der vorangegangenen Ansprüche, **gekennzeichnet, durch** ein Gehäuse (175), in dem die zumindest eine Trocknungsmittelkammer (110, 115) und die Sensorkammer (105) ausgebildet sind.

11. Detektionsvorrichtung (100) gemäß einem der vorangegangenen Ansprüche, **dadurch gekennzeichnet, dass** die Trocknungsmittelkammer (110, 115) das Trocknungsmittel (170) in pulver- oder kugelförmiger Form aufweist.

12. Verfahren (400) zum Detektieren zumindest eines Analyten (135) unter Verwendung einer Detektionsvorrichtung (100) gemäß einem der vorangegangenen Ansprüche, wobei das Verfahren (400) die folgenden Schritte aufweist:
- Einleiten (410) oder Aufnehmen des Analyten (135) in die Sensorkammer (105); und
- Pumpen (420) eines Mediums von der Sensorkammer (105) in die Trocknungsmittelkammer (110, 115) und/oder eines Mediums von der Trocknungsmittelkammer (110, 115) in die Sensorkammer (105), insbesondere um dem Medium Feuchtigkeit zu entziehen.

13. Computerprogramm, das dazu eingerichtet ist, das Verfahren (400) gemäß Anspruch 12 auszuführen und/oder anzusteuern.

14. Maschinenlesbares Speichermedium, auf dem das Computerprogramm nach Anspruch 13 gespeichert ist.
